(19) **Europäisches Patentamt**

**European Patent Office**

**Office européen des brevets**

(11) Publication number: **0 357 328**
**A1**

(12) # EUROPEAN PATENT APPLICATION

(21) Application number: **89308541.5**

(22) Date of filing: **23.08.89**

(51) Int. Cl.⁵: **C 07 C 19/08**
**C 07 C 17/38**

(30) Priority: **01.09.88 US 239568**

(43) Date of publication of application:
**07.03.90 Bulletin 90/10**

(84) Designated Contracting States:
**AT BE CH DE ES FR GB GR IT LI LU NL SE**

(71) Applicant: **E.I. DU PONT DE NEMOURS AND COMPANY**
**1007 Market Street**
**Wilmington Delaware 19898 (US)**

(72) Inventor: **Gehring, Douglas George**
**6 Alexander Drive**
**Wenonah New Jersey 08090 (US)**

**Merchant, Abid Nazarali**
**1408 Clive Circle**
**Wilmington Delaware 19803 (US)**

(74) Representative: **Woodcraft, David Charles et al**
**BROOKES & MARTIN High Holborn House 52/54 High Holborn**
**London, WC1V 6SE (GB)**

(54) **Purification of 2,2-dichloro -1,1,1-trifluoroethane.**

(57) A process for the purification of 2,2-dichloro-1,1,1-trifluoroethane comprising contacting crude 2,2-dichloro-1,1,1-trifluoroethane in the liquid state with an aqueous alkaline metal permanganate solution thereby to reduce the concentration of olefinic halocarbon contaminants.

## Description

### Purification of 2.2-Dichloro-1,1,1-trifluoroethane

#### Field of the Invention

A process for the purification of 2,2-dichloro-1,1,1-trifluoroethane comprising contacting crude 2,2-dichloro-1,1,1-trifluoroethane in the liquid state with an aqueous alkaline metal permanganate solution thereby to reduce the concentration of olefinic halocarbon contaminants.

#### Background of the Invention

Current concern over the depletion of the stratospheric ozone layer, particularly with the possible role of certain chlorofluorocarbons in accelerating such ozone depletion, has led to extensive investigation of alternative halocarbons which have reduced ozone depletion potentials.

One such alternative halocarbon is 2,2-dichloro-1,1,1-trifluoroethane ($CHCl_2CF_3$) which may be used in many of the applications where trichlorofluoromethane is currently used. Since 2,2-dichloro-1,1,1-trifluoroethane is a hydrogen-containing chlorofluorocarbon, it is expected to decompose sufficiently in the lower atmosphere and contribute little or nothing to ozone depletion in the stratosphere.

In the manufacture of 2,2-dichloro-1,1,1-trifluoroethane, impurities in the raw materials and the reaction conditions cause the resulting crude 2,2-dichloro-1,1,1-trifluoroethane to be contaminated with undesirable by-products, including olefinic halocarbons. Even the desired 2,2-dichloro-1,1,1-trifluoroethane can, under certain reaction conditions, undergo a small degree of dehydrohalogenation reaction to form undesirable olefinic halocarbons, as indicated by the following equation:
$$CHCl_2CF_3 \longrightarrow CCl_2 = CF_2 + HF$$
Other olefinic halocarbons which may be present in the crude 2,2-dichloro-1,1,1-trifluoroethane include 2-chloroperfluorobutene-2, chlorotrifluoroethylene and 2-chloro-1,1,1,4,4,4-hexafluorobutene-2.

These olefinic halocarbons, even when present in relatively small amounts, are objectionable since they are relatively unstable and are potentially toxic. If these olefinic halocarbon contaminants are not sufficiently removed from 2,2-dichloro-1,1,1-trifluoroethane, the use of 2,2-dichloro-1,1,1-trifluoroethane as a refrigerant, propellant, blowing agent, solvent and the like would be limited. However, because they are present in the crude 2,2-dichloro-1,1,1-trifluoroethane in relatively small amounts and because their boiling points are close to that of 2,2-dichloro-1,1,1-trifluoroethane, these olefinic halocarbons cannot be reduced conveniently to sufficiently low levels by commonly used fractional distillation procedures.

It is an object of the present invention to provide a process for the purification of crude 2,2-dichloro-1,1,1-trifluoroethane. It is a further object of the present invention to provide a purification process for crude 2,2-dichloro-1,1,1-trifluoroethane which is efficient and economical.

#### Summary of the Invention

According to the present invention there is provided a process for reducing the amount of olefinic halocarbons in 2,2-dichloro-1,1,1-trifluoroethane, said process comprising
contacting said 2,2-dichloro-1,1,1-trifluoroethane in the liquid state with an aqueous alkaline metal permanganate solution
separating liquid 2,2-dichloro-1,1,1-trifluoroethane from said aqueous metal permanganate solution and recovering purified 2,2-dichloro-1,1,1-trifluoroethane.

#### Details of the Invention

2,2-Dichloro-1,1,1-trifluoroethane is obtained by methods well known in the art including liquid or vapor phase hydrofluorination of perchloroethylene or pentachloroethane with HF, or by chlorination of 1,1,1-trifluoroethane or 1-chloro-2,2,2-trifluoroethane. As mentioned, in crude 2,2-dichloro-1,1,1-trifluoroethane there is usually present undesirable olefinic halocarbons including 1,1-dichlorodifluoroethylene, 2-chloroperfluorobutene-2, chlorotrifluoroethylene, and 2-chloro-1,1,1,4,4,4-hexafluorobutene-2. These olefinic halocarbons are usually present in amounts of from about 50 to about 1000 ppm (parts per million). Such amounts of olefinic halocarbons in 2,2-dichloro-1,1,1-trifluoroethane are unacceptable for its general use as a refrigerant, propellant and the like because the olefinic halocarbons are unstable and potentially toxic. Generally, the concentration of these olefinic halocarbon impurities in purified 2,2-dichloro-1,1,1-trifluoroethane should be less than about 10 ppm, more desirably less than 5 ppm.

The metal permanganate for treating the crude 2,2-dichloro-1,1,1-trifluoroethane should be dissolved in an aqueous alkaline solution. The metal permanganate may be alkali metal or alkaline earth metal permanganate, preferably alkali metal permanganate, more preferably sodium and potassium permanganate.

The concentration of metal permanganate can be any convenient value from about 1 weight percent to saturated aqueous solutions of about 200g per liter for sodium permanganate and about 60g per liter for potassium permanganate. It is convenient to use concentrations of from about 5 to about 6 weight percent of alkali metal permanganate.

The aqueous metal permanganate solution may be rendered alkaline by addition of an alkaline agent such as alkali metal hydroxide, alkali metal carbonate or alkaline earth metal hydroxide, preferably sodium or potassium hydroxide. For example, the alkali metal hydroxide may be conveniently added as an aqueous solution of from about 2 to about 10 weight percent metal hydroxide. The alkali agent need not be added to the metal permanganate solution directly but may be added to the crude 2,2-dichloro-1,1,1-trifluoroethane or to the mixture of it and the metal permanganate solution.

The relative volumetric proportions of 2,2-dichloro-1,1,1-trifluoroethane and the alkaline metal permanganate solution can vary considerably depending upon the concentrations of the olefinic halocarbons and of the metal permanganate solution, the degree of mixing of the two phases, the temperature to be used and the like. Generally, when using a metal permanganate solution of about 6 weight percent, a proportion of about 1:1 is suitable and convenient. In commercial operation, such a large stoichiometric excess would permit repeated use of the metal permanganate solution. For example, up to about 15 batches of crude 2,2-dichloro-1,1,1-trifluoroethane may be purified before the metal permanganate solution is depleted.

The temperature at which the crude 2,2dichloro-1,1,1-trifluoroethane is treated with the aqueous alkaline metal permanganate solution is not critical and may be any convenient temperature, for example, from about 5°C to about 70°C. Since 2,2dichloro-1,1,1-trifluoroethane boils at 27.6°C at atmospheric pressure, treatment at higher temperatures must be in a closed system to maintain it in a liquid state. The preferred temperature is from about 55°C to about 65°C.

The mixing of crude 2,2-dichloro-1,1,1-trifluoroethane and aqueous alkaline metal permanganate solution may be by any means known in the art such as shaking, agitation, plunging-jet mixing and the like. It is preferred that the two phases be intimately mixed.

Generally, after mixing the crude 2,2-dichloro-1,1,1-trifluoroethane with the aqueous alkaline metal permanganate solution, say at 60°C for about 2 to 4 hours, the olefinic halocarbon impurities will be reduced to desirable levels or eliminated. The mixing process can then be stopped and the mixture can be allowed to cool and to separate into two layers; the heavier 2,2-dichloro-1,1,1-trifluoroethane layer can then be recovered.

If desired, the treated 2,2-dichloro-1,1,1-trifluoroethane can be purified further, e.g., by fractional distillation. The process of the present invention is such that the olefinic halocarbon impurities in 2,2-dichloro-1,1,1-trifluoroethane are usually reduced to less than about 5 ppm (parts per million). The present process can be carried out batch-wise or continuously.

Example
Twenty ml (29.5g) of crude 2,2-dichloro-1,1,1-trifluoroethane which on gas chromatographic analysis indicated presence of 2-chloroperfluorobutene-2, chlorotrifluoroethylene, 1,1-dichlorodifluoroethylene and 2-chloro-1,1,1,4,4,4-hexafluorobutene-2 (200 ppm) was added to a "Teflon" bottle along with 25 ml of 6% aqueous potassium permanganate solution and 1 ml of 5% aqueous sodium hydroxide solution. The bottle was sealed, placed in a 60°C water bath and agitated vigorously for 2 hours. The bottle and its contents were allowed to cool to about 25°C, and the 2,2-dichloro-1,1,1-trifluoroethane layer was recovered and dried over anhydrous sodium sulfate. Gas chromatographic analysis indicated the following olefinic halocarbons to be present in the purified 2,2-dichloro-1,1,1-trifluoroethane in the amounts indicated:

| 2-chloroperfluorobutene-2 | less than 5 ppm |
| chlorotrifluoroethylene | less than 5 ppm |
| 1,1-dichloro-2,2-difluoroethylene | less than 5 ppm |
| 2-chloro-1,1,1,4,4,4-hexafluorobutene-2 | 6 ppm |

Claims

1. A process for reducing the amounts of olefinic halocarbons in 2,2-dichloro-1,1,1-trifluoroethane, said process comprising contacting said 2,2,-dichloro-1,1,1-trifluoroethane in the liquid phase with an aqueous alkaline metal permanganate solution, separating liquid 2,2-dichloro-1,1,1-trifluoroethane from said aqueous alkaline metal permanganate solution and recovering purified 2,2-dichloro-1,1,1-trifluoroethane.

2. A process according to claim 1 wherein said metal permanganate is sodium or potassium permanganate.

3. A process according to claim 1 or 2 wherein said contacting between 2,2-dichloro-1,1,1-trifluoroethane and the aqueous alkaline metal permanganate is carried out at a temperature from about 5°C to about 70°C.

4. A process according to claim 4 wherein said temperature is from about 5°C to about 65°C.

5. A process according to any one of the preceding claims in which the crude haloethane is mixed with the aqueous permanganate solution, then allowed to separate into two layers and the heavier haloethane recovered.

6. A process according to claim 5 wherein the mixing is conducted for about 2 to 4 hours.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 381 006 (I.C.I.) <br> * Claim 11 * <br> --- | 1-2 | C 07 C 19/08 <br> C 07 C 17/38 |
| X | US-A-4 129 603 (BELL) <br> * Claims * <br> --- | 1-3 | |
| X | DD-A- 233 839 (AKADEMIE DER WISSENSCHAFTEN DER DDR) <br> * Claims; examples * <br> --- | 1-2 | |
| X | US-A-3 388 175 (D.J. VINEY) <br> * Claims * <br> --- | 1-2 | |
| A | US-A-2 286 379 (H.A. ROBINSON) <br> ----- | 1 | |

TECHNICAL FIELDS
SEARCHED (Int. Cl.5)

C 07 C 17/00
C 07 C 19/00

The present search report has been drawn up for all claims

| Place of search | Date of completion of the search | Examiner |
|---|---|---|
| THE HAGUE | 22-11-1989 | VAN GEYT J.J.A. |

EPO FORM 1503 03.82 (P0401)